# EUROPEAN PATENT SPECIFICATION

(11) **EP 1 436 011 B1**
(45) Date of publication and mention of the grant of the patent: **03.10.2007**
(21) Application number: 02772526.6
(22) Date of filing: 17.10.2002
(51) Int. Cl.: A61K 47/48

(54) **DENDRIMERS FOR USE IN TARGETED DELIVERY**
DENDRIMERE ZUR VERWENDUNG IN GEZIELTER VERABREICHUNG
DENDRIMERES UTILISES POUR UNE ADMINISTRATION CIBLEE

(30) Priority: 19.10.2001 GB 0125216
(43) Date of publication of application: 14.07.2004
(73) Proprietor: University of Strathclyde, Glasgow G1 1XQ (GB); THE UNIVERSITY COURT OF THE UNIVERSITY OF GLASGOW, Glasgow, Lanarkshire G12 8QQ (GB)
(72) Inventor: UCHEGBU, Ijeoma, Florence, Glasgow G61 3DT (GB); MUNRO, Avril, Anstruther, Fife KY10 2AW (GB); SCHATZLEIN, Andreas, Gerhart, Glasgow G61 3DT (GB); GRAY, Alexander, Irvine, Glasgow G44 3NL (GB); ZINSELMEYER, Bernd, D-33129 Delbrueck (DE)
(74) Representative: Kiddle, Simon John
(86) International application number: PCT/GB2002/004706
(87) International publication number: WO 2003/033027

(56) References cited:
- WO-A-01/76633
- WO-A-94/23759
- WO-A-98/40502
- WO-A-03/001218
- US-A- 5 714 166
- US-B1- 6 440 743
- GEBHART C L ET AL: "Evaluation of polyplexes as gene transfer agents." JOURNAL OF CONTROLLED RELEASE : OFFICIAL JOURNAL OF THE CONTROLLED RELEASE SOCIETY. 15 JUN 2001, vol. 73, no. 2-3, 15 June 2001 (2001-06-15), pages 401-416, XP002246815 ISSN: 0168-3659
- KABANOV, V. A. ET AL: "Interpolyelectrolyte Complexes Formed by DNA and Astramol Poly(propylene imine) Dendrimers" MACROMOLECULES (2000), 33(26), 9587-9593 CODEN: MAMOBX; ISSN: 0024-9297, 26 December 2000 (2000-12-26), XP002246835
- ZINSELMEYER B H ET AL: 'The lower-generation polypropylenimine dendrimers are effective gene-transfer agents' PHARMACEUTICAL RESEARCH 2002 UNITED STATES vol. 19, no. 7, 2002, pages 960 - 967, XP008019182 ISSN: 0724-8741

## Description

The present invention relates to the targeted delivery of bioactive molecules in a mammalian body. In particular, the present invention relates to the use of cationic dendrimers for delivering polynucleotide molecules, peptides and polypeptides and/or pharmaceutical agents to a mammalian body, in particular, human.

The possibility of using genes as medicines to correct genetic disorders or treat cancers is hampered by the inability to efficiently deliver genetic material to diseased sites¹. A variety of viral and non-viral systems are being used experimentally each with distinct advantages and disadvantages. Viral systems² have been studied extensively and include a wide variety of viral types such as, retroviruses, adenoviruses, adeno-associated viruses, herpes simplex virus and the HIV based lentivirus. All have various inherent disadvantages³ such as safety concerns and scale-up difficulties. Non-viral systems such as cationic liposomes⁴⁻⁶, cationic polymers^{7,8}, cationic polymeric vesicles^{9,10} and dendrimers¹¹⁻¹⁴ have thus been studied as gene delivery agents in an effort to circumvent some of the safety and production problems associated with viruses. As well as applications in human health, suitable gene transfer systems are commercially attractive as in vitro molecular biology and in vivo transfection reagents for laboratory use.

Dendrimers are synthetic 3-dimensional macromolecules that are prepared in a stepwise fashion from simple branched monomer units, the nature and functionality of which can be easily controlled and varied. Dendrimers are synthesised from the repeated addition of building blocks to a multifunctional core (divergent approach to synthesis), or towards a multifunctional core (convergent approach to synthesis) and each addition of a 3-dimensional shell of building blocks leads to the formation of a higher generation of the dendrimers⁵¹. Polypropylenimine dendrimers start from a diaminobutane core to which is added twice the number of amino groups by a double Michael addition of acrylonitrile to the primary amines followed by the hydrogenation of the nitriles⁵². This results in a doubling of the amino groups⁵².

Polypropylenimine dendrimers contain 100% protonable nitrogens¹⁷ and up to 64 terminal amino groups (generation 5, DAB 64)^{15,16}. Protonable groups are usually amine groups which are able to accept protons at neutral pH. The use of dendrimers as gene delivery agents has largely focused on the use of the polyamidoamine^{11-13,18-25} and phosphorous containing¹⁴ compounds with a mixture of amine/ amide or N-P(O₂)S as the conjugating units respectively with no work being reported on the use of the lower generation polypropylenimine dendrimers for gene delivery. Polypropylenimine dendrimers have also been studied as pH sensitive controlled release systems for drug delivery^{26,27} and for their encapsulation of guest molecules when chemically modified by peripheral amino acid groups²⁸. The cytotoxicity²⁹ and interaction of polypropylenimine dendrimers with DNA³⁰ as well as the transfection efficacy of DAB 64 has also been studied³¹.

Kabanov and others report that polypropylenimine dendrimers interact with DNA via the surface primary amines only with no involvement of the internal amine groups³⁰ while Gebhart and Kabanov report very low gene transfer activity with the 5^{th} generation polypropylenimine dendrimers DAB 64 in the easy to transfect COS cell line³¹. These workers also report that DAB 64 is far too toxic above a dendrimer. DNA weight ratio of 0.62: 1 (nitrogen to phosphate ratio of 4: 1). Additionally Malik and others conclude that the cationic dendrimers as opposed to the anionic dendrimers are too toxic for parenteral use without further derivatisation with biocompatible groups such as polyethylene glycol units²⁹.

US 5,714,166 discloses dendrimers and their uses as carrier materials.

The present invention is based upon the observation that, contrary to earlier reports, cationic dendrimers, such as polypropylenimine dendrimers, display suitable properties, such as specific targeting and low toxicity, for use in the targeted delivery of bioactive molecules, such as genetic material. In addition, derivatives of the cationic dendrimer also display suitable properties for the targeted delivery of bioactive molecules.

The chemical modification (derivatisation) of polypropylenimine dendrimers has been extensive with reports on the conjugation of amino acid^{28,32}. carboxylate^{33,34}, acetyl³⁵, 2-(hydroxy)propyltrimethylammonium²⁷, dimethyldodecylammoniumm³⁶, 3,4,5-(ologoethylenoxy) benzoyl³⁷, alkanoyl thioglucose³⁸, thiolactosyl³⁹ groups as well as the conjugation of hydrophobic 3,4-bis(decyloxy)benzoyl⁴⁰, palmitoyl⁴¹, pentafluorphenyl 11-[4-(4-hexyloxyphenylazo)phenyloxy]undecanoyl⁴¹, adamantine carboxyl⁴¹, decanoyl⁴², dodecyl⁴², polyisobutylene⁴³, stearoyl⁴⁴, ω-(4'cyanobiphenyloxy)alkyl⁴⁵, and oligo(p-phenylene vinylene)⁴⁶ groups to the primary amino groups on the surface of polypropylenimine dendrimers.

In addition to the targeted delivery of genetic material, selective targeting of other bioactive agents, such as peptides/polypeptides and pharmaceutical agents, is sought.

It is thus one of the objectives of the present invention to provide cationic dendrimers capable of targeted delivery of bioactive molecules to a particular location in a mammalian body.

Accordingly, the present invention provides a said composition comprising a cationic polypropylenimine dendrimer comprising a diaminobutane core with 1, 2 or 3 generations of propylenimine molecules attached which is admixed with the bioactive molecule, wherein the cationic dendrimer is optionally modified by covalently binding derivatising groups.

The term "cationic dendrimer" refers to a dendrimer molecule which possesses a positive charge at physiological pH. However, the dendrimer derivatives of the present invention may not in themselves be cationic as a result of the derivatisation.

The cationic dendrimers, or derivatives thereof, of the composition of the present invention may be derived from a core molecule comprising 2 to 10 carbon atoms, such as 3 or 4 carbon atoms, and in particular 4 carbon atoms with one or more functional groups which may, for example, be amine groups, It will be appreciated that, for example, the cationic dendrimers, or derivatives thereof, may be derived from a core molecule such as diaminoethane, diaminopropane or diaminobutane, and in particular, diaminobutane.

The groups attached to the core molecule may, for example, include propylamines Thus, the dendrimers may be polypropylenimine dendrimers, or derivatives thereof, and may possess a diaminobutane core.

The term "polypropylenimine dendrimer" is intended to refer to dendrimers comprising a diaminobutane core with 1, 2, 3, 4 or 5 generations of propylenimine molecules attached. The term encompasses DAB 4, DAB 8, 16, 32 and 64, DSAM 4, DSAM8, 16, 32 and 64, and QDAB4, 8, 16, 32 and 64, HDAB4, 8, 16, 32, 64 and bolamphiphilic polypropylenimine dendrimers BDAB4, BDAB8, BDAB16, BDAB32, BDAB64. Although all generations of the compounds DSAM, QDAB, HDAB and BDAB comprise a diaminobutane core with propylenimine groups attached thereto, the term "DAB" as generally used herein is intended to refer to the underivatised DAB8, 16, 32 and 64 compounds, unless otherwise indicated,

The term "generation" refers to the number of iterative reaction steps that are necessary to produce the compound. The number which follows the name or abbreviated name of the dendrimer, for example, 8, 16, 32 or 64 refers to the number of surface groups on the dendrimer molecule itself, which amino groups may or may not be derivatised.

The cationic dendrimers of the composition of the present invention may be modified by covalently binding derivatising groups, such as hydrophobic, hydrophilic or amphiphilic groups to the surface of the dendrimer or by attaching two dendrimer molecules to either end of a hydrocarbon chain with a carbon length of 8, 12, 14, 16 or 18 carbons to give bolamphiphilic dendrimers (said modified dendrimers referred to herein as "derivatives"). The number of derivatising groups may vary from one derivatising group per dendrimer molecule up to and including derivatising all available surface or terminal groups on the dendrimer molecule, for example, derivatising all 16 surface groups of the DAB16 molecule.

The amphiphilic derivative comprises a hydrophilic and a hydrophobic segment. The hydrophilic segment may be derived from a phosphoglycerate molecule, for example, glycerol 3-phosphate. The hydrophobic segment is covalently bound to the hydrophilic segment, for example, via an ester linkage. The hydrophobic segment is selected from any suitable hydrophobic group, for example, alkyl, alkenyl or alkynyl groups of 8-24 carbons in length. Therefore, the hydrophobic segment plus ester linkage can be defined as an acyl group. The amphiphilic derivative is attached to the dendrimer by a linker molecule, such as polyethylene glycol (PEG) or a sugar unit such as muramic acid bound to the hydrophilic segment. The length of the PEG linker molecule may for example be in the range of 1 to 120 ethylene glycol units, for example 50-100 and, for example, 70-80, for example, 77. In particular the linker molecule may be polyethylene glycol with a Mw of approximately 3,500. Alternatively, the linker molecule may be an ester, amine or ether linkage for ordinary hydrophobic modifications or a sugar molecule such as muramic acid. In particular, the derivative may be a phosphoglyceride such as a phosphatidyl ethanolamine, for example, distearoylphosphatidylethanolamine. The number of amphiphilic derivatives per dendrimer molecule may range from 1 derivatising group per dendrimer molecule to derivatising all of the groups of the dendrimer (the generation of the dendrimer will determine the total number of surface groups available for derivatising), and may be, in particular, one group per dendrimer. Thus, the dendrimers of the present invention include generations 1, 2, 3, 4 and 5 of the amphiphilic-derivatised diaminobutane dendrimer referred to herein as 1,2-diacyl-SN-glycero-3-phosphoethanolamine-N-[(polyethyleneglycol)-N-diaminobutanepolypropylenimine dendrimer -(NH₂)ₓ], where x = 4, 8, 16, 32 or 64.(conveniently referred to as DSAM4, 8, 16, 32 or 64, respectively).

The hydrophobic derivative may be an alkyl, acyl, alkenyl, alkynyl or aryl group of 8-24 carbons in length. It is to be understood that the term "hydrophobic" can encompass acyl groups when the chain length of such acyl groups is 8 carbons or more and may, for example, be a hexadecanoyl group. The number of hydrophobic groups per dendrimer molecule may range from 1 derivatising group per dendrimer molecule to derivatising all of the groups of the dendrimer (the generation of the dendrimer will determine the total number of surface groups available for derivatising), and may be, in particular, one group per dendrimer. Thus, the dendrimers of the present invention include generations 1,2, 3, 4 and 5 of the hydrophobic - derivatised diaminobutane dendrimer referred to herein as HDAB4, HDAB8, HDAB16, HDAB32, HDAB64.

The bolamphiphiles may consist of two molecules of any of the dendrimers DAB4, DAB8, DAB 16, DAB 32, DAB 64 linked to either end of an alkyl, acyl, alkenyl, alkynyl hydrophobic unit of 8 to 24 carbon chains in length or alternatively linked by an aryl group and may be a C12 bolamphiphile of DAB 4 or DAB 8. The term "bolamphiphiles" is understood to refer to an amphiphilic molecule wherein the hydrophilic groups are separated by the hydrophobic groups. Thus, the dendrimers of the present invention include C8-C16alkyl bolamphiphiles of dendrimers of generations 1,2, 3, 4 and 5 herein referred to as B8DAB4, 8, 16, 32 or 64; B10DAB4, 8, 16, 32 or 64; B12DAB4, 8, 16, 32 or 64, B14DAB4, 8, 16, 32 or 64 and B16DAB4, 8, 16, 32 or 64 . The amino derivative may, for example, be a tertiary amine or quaternary ammonium derivative, and in particular a quaternary derivative comprising C1-C4 alkyl groups, such as 3 methyl groups, covalently bound to a nitrogen atom on the surface of the dendrimer. The number of ammonium derivatives per dendrimer molecule may range from 1 derivatising group per dendrimer molecule to derivatising all groups of the dendrimer (the generation of the dendrimer will determine the total number of surface groups available for derivatising), and may be, in particular, all groups available for derivatising. Thus, the dendrimers of the present invention include generations 1, 2, 3, 4 and 5 of the quaternary ammonium-derivatised diaminobutane dendrimer referred to herein as quaternary ammonium diaminobutanepolypropylenimine dendrimer -[NH₂(CH₃)₃]ₓ, where x = 4, 8, 16, 32 or 64. (conveniently referred to as QDAB4, 8, 16, 32 or 64, respectively).

The dendrimers in the present invention may also be derivatised with hydrophilic groups such as sugars, mono and oligohydroxy C1-C6 alkyl, mono and oligohydroxy C2-C6 acyl, C1-C2 alkoxy alkyl optionally having one or more hydroxy groups substituted on the alkoxy or alkylene groups, amino acids, peptides of 1-200 amino acids in length and oligo or poly-(oxa C1-C3 alkylene) such as polyoxyethylene comprising 1-120 ethylene oxide units.

Target locations for the delivery of bioactive molecules include the liver, spleen, lung, kidney and heart. In particular, two of the dendrimers of the present invention studied, DAB16 and DSAM16, have displayed organ-specific targeting to the liver and spleen, respectively.

Therefore, the present invention also provides a composition for the delivery of a bioactive molecule to the liver of a recipient, wherein said composition comprises the polypropylenimine dendrimer DAB16 admixed with a said bioactive molecule. Additionally, the present invention provides a composition for the delivery of bioactive molecules to the spleen of a recipient, wherein said composition comprises the polypropylenimine dendrimer DSAM16 admixed with a said bioactive molecule.

The recipient may be a mammal, such as a human.

The terms "bioactive molecules" and "biologically active molecules" are intended to encompass polynucleotides, peptides/polypeptides and/or pharmaceutical agents. The term "polynucleotides" generally refers to DNA unless otherwise indicated but may include RNA, cDNA, oligonucleotides, plasmids etc. The term may also be used interchangeably herein with the terms "polynucleotide", "gene", "genetic material" and "genetic sequence". Such genes intended for expression are common to the field of gene therapy and include, but are not limited to, sense DNA or RNA for expressing a product in the target organ, or antisense DNA or RNA for reducing or eliminating expression of a native or introduced gene in the target organ. The term "peptide" refers to a chain of 4 to 600 amino acids long, such as 4 to 200 amino acids long and therefore encompasses polypeptides and proteins, and includes enzymes and polypeptide hormones. Furthermore, peptides modified by, for example, glycosylation, are also included in the present invention, as is a protein comprising two or more polypeptide chains each of length of 4 to 600 amino acids long cross-linked by, for example, disulphide bonds, for example, insulin and immunoglobulins. The term "pharmaceutical agent" is intended to include any natural or synthetic compound administered to a recipient in order to induce a physiological or pharmacological effect. Examples of such agents are anti-tumour drugs, antibiotics, hormones, anti-inflammatory agents, antiparasitic agents, DNA vaccines, etc

The cationic dendrimers are admixed with the bioactive agents in preparing the compositions of the present invention for delivery. The term "admixed" generally refers to the bioactive agent being associated with but not covalently bound to the dendrimer. The term is however also intended to encompass covalently binding the bioactive agent to the dendrimer via any suitable reactive group on the dendrimer and the agent.

Where the bioactive agent is a polynucleotide molecule, the molecule is usually associated with, that is, not covalently bound to, the dendrimer to allow the polynucleotide to be expressed. However, it may also be possible that expression of a covalently bound polynucleotide molecule can occur, and therefore, these covalently bound polynucleotide molecules are intended to be encompassed by the present invention.

In a yet further aspect, the present invention provides a pharmaceutical formulation comprising a composition of the present invention, and a pharmaceutically acceptable carrier.

Pharmaceutically acceptable carriers are well known to those skilled in the art and include, but are not limited to, 0.1 M and preferably 0.05M phosphate buffer or 0.8% (w/v) saline. Additionally, such pharmaceutically acceptable carriers may be aqueous or non-aqueous solutions, suspensions, and emulsions. Examples of non-aqueous solutions are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

Conveniently, the composition or pharmaceutical formulation of the present invention may include an agent which assists in forming a colloidal suspension, for example, 5% dextrose solution. Other agents which may be included are viscosity enhancing polymers such as alginates and polyethyleneglycol polymers, buffering agents and mixtures of aqueous and non-aqueous solvents in emulsions.

The present invention also provides the use of the composition or pharmaceutical formulation of the present invention for the preparation of a medicament for delivery or bioactive molecules to a target location in the body of a recipient.

Although the dendrimers of the present invention display suitable properties for the delivery of bioactive molecules in vivo, they are also useful for transfecting mammalian cells in vitro. Thus, the present invention also provides a composition of the present invention for transfecting mammalian cells with a bioactive molecule in vitro. The mammalian cells may, for example, be human cells.

The present invention still further provides 1,2-diacyl-SN-glycero-3-phosphoethanolamine-N-[(polyethyleneglycol)-N-diaminobutanepolypropylenimine dendrimer-(NH₂)ₓ], where x = 4, 8, 16, 32 or 64 (DSAM), and quaternary ammonium diaminobutanepolypropylenimine dendrimer -[NH₂(CH₃)₃]ₓ, where x = 4, 8. 16, 32 or 64 (QDAB). The DSAM or QDAB dendrimers may, for example, be second, third, fourth or fifth generation dendrimers referred to herein as DSAM4, 8, 16, 32 and 64, and QDAB4, 8, 16. 32 and 64.

The present invention also provides a method of preparing a composition as described above, said method comprising admixing a cationic dendrimer, and/or derivatives thereof, and a bioactive molecule.

These and other aspects of the present invention will now be described by way of example only, in conjunction with the accompanying Figures, in which:

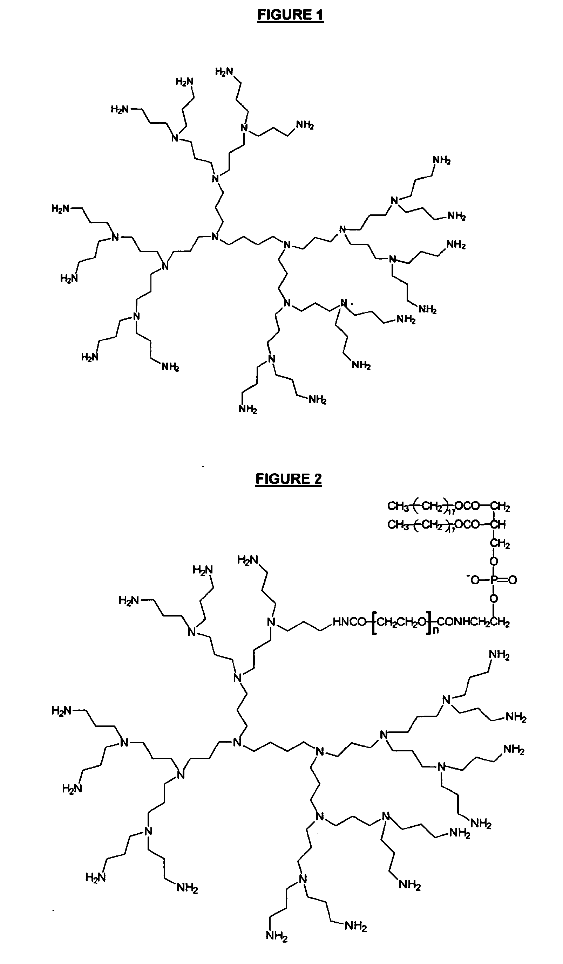

Figure 1 illustrates DAB 16 generation 3 polypropylenimine dendrimer, DAB 64 contains 2 more generations of propylamines attached to this molecule;
Figure 2 illustrates DSAM 16, an amphiphilic derivative of DAB 16 (DSPE-PEG-NHS + DAB16);
Figure 3 illustrates BnDAB4, a bolamphiphile derivative of DAB4 (n= 8, 10 or 12 to give B8DAB4, B10DAB4, B12DAB4 respectively);

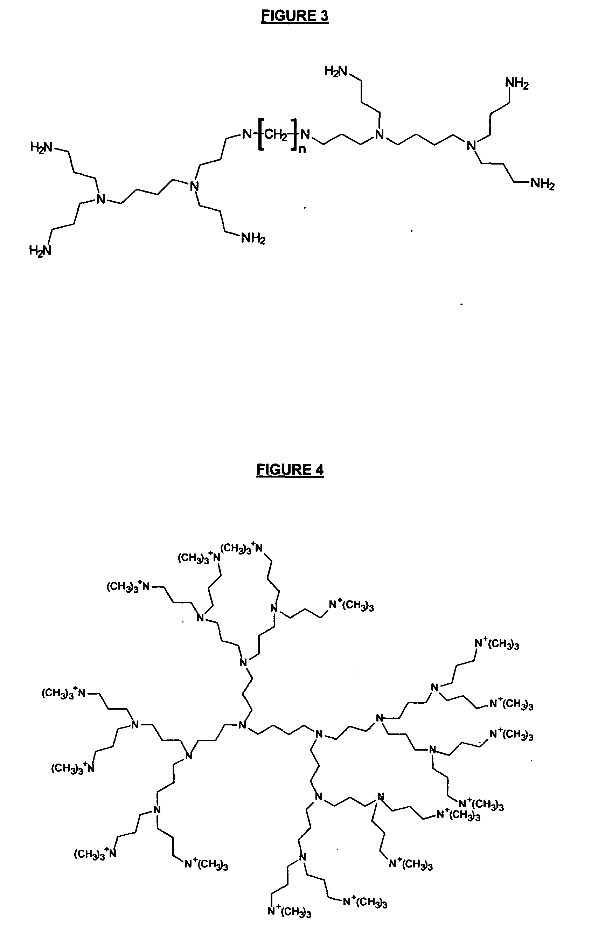

Figure 4 illustrates QDAB 16, a quaternary ammonium derivative of DAB 16 (CH₃l + DAB16);
Figure 5 illustrates Luciferase gene expression in vivo;
Figure 6 illustrates liver targeting of gene expression by the polypropylenimine dendrimers; and
Figure 7 illustrates tumour gene expression after the intravenous administration of DNA, PEI-DNA = the Exgen 500 formulation.

### EXAMPLE 1

### METHODS

### Synthesis of modified dendrimers

### DSAM

To DAB 16 (Sigma Aldrich, Dorset, UK - 3.52g, 1.32mmoles) dissolved in absolute ethanol (70ml) was added triethylamine (27ml, 6.6mmoles) and to this solution was added dropwise DSPE-PEG-NHS (distearoylphosphatdylethanolamine polyethylene glycol N-hydroxysuccinimide - 500mg, 0.11mmoles, Shearwater Polymers) dissolved in chloroform (25mL) over 60 minutes. The reaction was left stirring protected from light for 72h. At the end of this time the reaction mixture was evaporated to dryness by rotary evaporation under reduced pressure at 50°C. The residue was redissolved in absolute ethanol (40ml), filtered and the filtrate evaporated to dryness. This latter residue was dissolved in water (80ml) and dialysed against 5 l of water over 24h with 6 changes. The dialysate was freeze-dried and the structure confirmed by ¹H and ¹³C NMR.

### QDAB

DAB 16, 32 or 64 (500mg) was dispersed in methyl-2-pyrolidone (50ml) for 16h at room temperature with stirring. To the DAB dispersion was added sodium hydroxide (120mg), methyl iodide (3g) and sodium iodide (150mg). The reaction mixture was stirred under a stream of nitrogen for 3h at 36°C. The quaternary ammonium product was recovered by precipitation in diethyl ether followed by filtration. The solid was washed with copious amounts of absolute ethanol (1l) followed by copious amounts of diethyl ether (500ml). The washed solid was then dissolved in water (150ml) and passed over an ion exchange column (1 x 6cm packed with 30ml Amberlite IRA-93 Cl⁻ and subsequently washed with HCl - 90ml, 1M followed by distilled water - 500ml until the eluate gives a neutral pH). The eluate obtained was freeze-dried and the structure confirmed by both ¹H and ¹³C NMR.

### DNA condensation

Plasmid (pCMVsport β-gal or pCMV luciferase, Life Technologies, UK) was grown in E. coli and plasmid purification carried out using a QIAGEN Endo-toxin free Giga Plasmid Kit (QIAGEN, Hilden, Germany) according to the manufacturer's instructions. Purity was confirmed by agarose gel electrophoresis⁴⁷. The reduced fluorescence of ethidium bromide (EthBr) was used to probe for DNA condensation by the polymers. EthBr fluorescence increases significantly (factor 40 compared to unbound EthBr) on intercalation with double stranded DNA⁴⁸. The electrostatic interaction between the anionic DNA and cationic groups of the carrier on formation of the DNA - vesicle complex reduces the number of EthBr binding sites, a process termed condensation, ultimately reducing the fluorescence intensity of the EthBr solution.

Complexes of DAB 16 and DSAM with DNA were prepared at various polymer, DNA weight ratios and at various time points the fluorescence intensity (λ_{excitation} = 526nm, λₑₘᵢₛₛᵢₒₙ. = 592nm) of the complexes determined in the presence of EthBr (40µg ml⁻¹). The DNA concentration in the cuvette was kept constant (100µg ml⁻¹) and the polymer solutions in PBS (phosphate buffered saline, pH = 7) and a solution of DNA in PBS served as controls. The reduced fluorescence (Fₜ/ F₀), was determined for each of the samples, where Fₜ = the fluorescence of the DNA, polymer complexes and F₀ = the fluorescence of DNA alone.

### In vitro cytotoxicity assay

A human epidermoid carcinoma cell line (A431, ATCC CRL-1555) was maintained in Dulbecco's minimum essential medium (DMEM) supplemented with 10% foetal calf serum (FCS) and 2mM glutamine (GibcoBRL, UK) at 10% CO₂ and 37°C.

Polypropylenimine dendrimer/ dendrimer derivative formulation cytotoxicity was assessed by the measurement of the IC50 in a standard MTT (3-[4,5-dimethylthiazol-2-yl]-2,5-diphenyltetrazolium bromide thiazolyl blue - indicator dye) assay⁴⁹. Briefly, 96 well microtitre plates were seeded with 5000 cells per well and incubated for 24hrs. Dilutions of the dendrimer/dendrimer - DNA formulations (100µl) in tissue culture medium (Opti-Mem) were incubated with the cells for 4 h. The samples were then replaced with fresh DMEM daily and incubated for 72 h. After this period the indicator dye (50µl, 50mg ml⁻¹) was added to each well and incubated with the cells for 4h in the dark. The medium and indicator dye were then removed and the cells lysed with dimethylsulphoxide (200µl). After addition of Sorensen's glycine buffer (25µl) the absorption was measured at 570nm. Values were expressed as a percentage of the control to which no vesicles were added.

### In vitro transfection

### DNA polymer formulations

DAB and QDAB dendrimer - DNA (pCMVSport β-galactosidase) formulations were made by mixing DNA and dendrimers in a 5% dextrose solution and allowing to stand for no longer than 15 minutes before use. The resulting colloidal dispersion was sized by photon correlation spectroscopy (Malvem Instruments, UK).

### Cell culture

A431 cells (human epidermoid carcinoma cell line, ATCC, CRL-1555), maintained in Dulbecco's Minimal Essential Medium (DMEM, Life Technologies, UK) supplemented with foetal calf serum and L-glutamine (2mM) were seeded at a density of 10⁴ cells ml⁻¹ and 200µL of the cell suspension placed in 96 well flat bottomed plates. Cells were incubated for 24h at 37°C in 10% CO₂. Polymer - DNA complexes containing 200µg m┌¹ DNA (100µl) and serum free medium (100µl, OPTIMEM, Life technologies, UK) were incubated with the cells for 4h at 37°C in 10% CO₂. Naked DNA served as the negative control while a formulation comprising N-[1-(2,3-dioleoyloxy)propyl]-N,N,N-trimethylammonium methylsulphate (DOTAP), DNA (5:1) served as the positive control. Both the negative and positive controls were dosed at a level of 20µg DNA per well while the level of DNA dosed with the dendrimers varied as indicated. After this time the incubation medium was replaced with DMEM culture media containing penicillin (100U ml⁻¹) and streptomycin (0.1mg ml⁻¹) and once again incubated at 37°C in 10% CO₂ for 48h. The cells were then washed in phosphate buffered saline (200µl) and lysed with 1x Passive Lysis Buffer (80µL, Promega, UK) for 30 min. The cell lysates were subsequently analysed for β-galactosidase expression as described below.

### β-galactosidase expression

To the assay buffer [50µl (sodium phosphate buffer - 200mM, pH = 7.3, magnesium chloride - 2mM, mercaptoethanol - 100mM, o-nitrophenol-β-galactopyranoside - 1.33mg ml⁻¹)] was added an equal volume of the cell lysate from above within 96 well flat-bottomed plates. Samples were incubated for 1h and the visible absorbance read at 405nm on an automatic plate reader.

### In vivo transfection

### DNA-polymer formulations

Exgen 500 (linear polyethylenimine 22kD, Euromedex, France) was formulated with the luciferase reporter gene (pCMV luciferase) as described by the manufacturers. Both DAB 16 and DSAM were dissolved in 5%w/v dextrose by probe sonication (5 minutes with the instrument set at 15% of its maximum output) and mixed in a 5:1 weight ratio with DNA 15 minutes prior to intravenous injection.

### Animal Experiments

Groups of Balb/C mice (n = 3) were injected via the tail vein with either the DAB 16, DSAM, Exgen 500 or naked DNA formulations each containing 100µg of DNA in an injection volume of 200 - 400µl. Exgen 500 and naked DNA served as the positive and negative controls respectively. Organs were harvested 24h later and quickly frozen in liquid nitrogen and stored at -80°C until analysis could be performed on them.

### Analysis for Luciferase

To the liver samples were added 5µl of tissue lysis buffer (25mM Tris HCl - pH = 8.0, 2mM DTT, 2mM EDTA - pH 8.0, 10%w/v glycerol, 1%w/v Triton X-100, 1 Complete^{®} protein inhibitor cocktail tablet per 50 ml of buffer) per mg of liver tissue. To the other organs was added 4µl of tissue lysis buffer per mg of tissue. Organs were homogenised on ice in the lysis buffer to form a slurry and the slurry incubated on ice for 15 mins. The resulting slurry was centrifuged at 13,000rpm to pellet tissue debris and the supernatant removed to a fresh tube. To the pellet was added another 50µl of lysis buffer. The pellet was resuspended by homogenisation and centrifuged once more at 13,000rpm and the supernatant removed and added to the previous supernatant sample. Supernatant samples were diluted 1:10 with the tissue lysis buffer and 80µl of this solution sampled and used for the luciferase assay which was carried out according to the protocol provided by Promega, UK. Protein estimation was carried out using the Sigma (Sigma-Aldrich, UK) bicinchoninic acid system (BC-1).

### RESULTS AND DISCUSSION

DNA binds electrostatically with the nitrogen rich dendrimers DAB 16, DAB 32 and DAB 64 (Table 2) and presumably with DAB 8. These compounds begin to condense DNA at a nitrogen to phosphate ratio of 1.47, 1.49 and 1.45 respectively (Table 1) and a surface nitrogen to phosphate ratio of 0.78, 0.77 and 0.74 respectively.

**Table 1: % Condensation of DNA by DAB Dendrimers**

| **Dendrimer** | **Dendrimer, DNA weight ratio** | **% DNA condensation after 30 min** |
|---|---|---|
| DAB 16 | 5.00 | 90.3 |
| | 2.00 | 93.9 |
| | 1.00 | 96.2 |
| | 0.50 | 96.0 |
| | 0.25 | 94.1 |
| | 0.13 | 42.8 |
| QDAB 16 | 5.00 | 96.2 |
| | 2.00 | 95.3 |
| | 1.00 | 95.7 |
| | 0.50 | 94.6 |
| | 0.25 | 33.6 |
| | 0.13 | 19.2 |
| DAB 32 | 5.00 | 94.4 |
| | 2.00 | 96.9 |
| | 1.00 | 96.7 |
| | 0.50 | 96.1 |
| | 0.25 | 87.2 |
| | 0.13 | 21.8 |
| QDAB 32 | 5.00 | 96.7 |
| | 2.00 | 96.9 |
| | 1.00 | 96.4 |
| | 0.50 | 43.9 |
| | 0.25 | 12.6 |
| | 0.13 | 7.5 |
| DAB 64 | 5.00 | 93.4 |
| | 2.00 | 95.4 |
| | 1.00 | 93.1 |
| | 0.50 | 96.2 |
| | 0.25 | 87.9 |
| | 0.13 | 26.3 |
| QDAB 64 | 5.00 | 96.0 |
| | 2.00 | 96.2 |
| | 1.00 | 95.4 |
| | 0.50 | 46.0 |
| | 0.25 | 15.3 |
| | 0.13 | 11.9 |
| DSAM 16 | 5 | 85.1 |
| | 10 | 85.0 |
| | 15 | 85.9 |
| | 20 | 85.7 |

It appears as if DNA does not bind only to the surface nitrogens of polypropylenimine dendrimers as reported³⁰ but also to nitrogens possibly in the second shell of the dendrimer also. Condensation of DNA with the polyamidoamine dendrimers has also been found to occur by electrostatic means¹⁹. We propose that the polypropylenimine dendrimers have advantages over the polyamidoamine polymers for gene delivery applications simply due to the increased content of protonable nitrogens on the polypropylenimine polymers. There are also advantages associated with the -polymer shape over linear polymers as DNA appears to interact with the surface primary amines only, leaving the internal tertiary amines available for the neutralization of the acid pH⁵⁰ within the endosomal/ lysosomal compartment. The release of polyamidoamine carried genes by the endosome has been attributed to the protonation of the internal tertiary nitrogens by endosomal protons which then results in a swelling of the endosome and the release of the DNA to the cytoplasm¹². Also the hydrolytic degradation of polyamidoamine dendrimer amide bonds in water or ethanol^{12, 13} increases transfection efficacy up to 50 fold which the authors attribute to the increased flexibility of the polymer on heat degradation. This increased flexibility is said to be crucial to the swelling of the endosome¹² . However we propose that simply increasing the level of tertiary amines in the polymer available for neutralization of the endosomal/ lysosomal pH improves transfection irrespective of the flexibility of the polymer.

In vitro transfection efficacy with the polypropylenimine dendrimers reveals that protein expression obtained by using 20µg of DNA in the DOTAP formulation is obtained using just 5ug of DNA in the DAB 16 formulation and there is no significant difference between the use of 15µg DNA in the QDAB 16 formulation and 20µg DNA in the DOTAP formulation (Table 2).

**Table 2: The in vitro transfection of DAB dendrimers in the A431 cell line**

| **Formulation** | **DNA dose per well (µg)** | **β-galactosidase expression*** |
|---|---|---|
| DAB 8, DNA (3: 1 g g⁻¹) | 20 | 101.3 |
| | 15 | 102.6 |
| | 10 | 80.1 |
| | 5 | 45.1 |
| DAB 8, DNA (5: 1 g g⁻¹) | 20 | 108.7 |
| | 15 | 96.5 |
| | 10 | 73.1 |
| | 5 | 59.2 |
| DAB 8, DNA (10: 1 g g⁻¹) | 20 | 82.0 |
| | 15 | 76.4 |
| | 10 | 78.6 |
| | 5 | 71.7 |
| DAB 16, DNA (1: 1 g g⁻¹) | 20 | 54.2 |
| | 15 | 40.0 |
| | 10 | 38.3 |
| | 5 | 33.7 |
| DAB 16, DNA (3: 1 g g⁻¹) | 20 | 23.9 |
| | 15 | 40.7 |
| | 10 | 57.7 |
| | 5 | 56.5 |
| DAB 16, DNA (5: 1 g g⁻¹) | 20 | 18.3 |
| | 15 | 24.1 |
| | 10 | 88.4 |
| | 5 | 100.8 |
| QDAB 16, DNA (1: 1gg⁻¹) | 20 | 54.2 |
| | 15 | 40.0 |
| | 10 | 38.3 |
| | 5 | 33.7 |
| QDAB 16, DNA (3: 1 g g⁻¹) | 20 | 76.4 |
| | 15 | 70.4 |
| | 10 | 45.3 |
| | 5 | 13.8 |
| QDAB 16, DNA (5: 1 g g⁻¹) | 20 | 42.3 |
| | 15 | 50.8 |
| | 10 | 48.5 |
| | 5 | 45.5 |
| DSAM 16, DNA (5: 1g g⁻¹) | 20 | 28.6 |
| | 15 | 35.2 |
| | 10 | 25.1 |
| | 5 | 26.7 |
| DSAM 16, DNA (10: 1 g g⁻¹) | 20 | 26.1 |
| | 15 | 22.2 |
| | 10 | 22.3 |
| | 5 | 24.3 |
| DSAM 16, DNA (15: 1 g g⁻¹) | 20 | 21.1 |
| | 15 | 21.6 |
| | 10 | 21.4 |
| | 5 | 21.9 |
| DAB 32, DNA (3: 1 g g⁻¹) | 20 | 12.2 |
| | 15 | 16.3 |
| | 10 | 17.6 |
| | 5 | 16.6 |
| QDAB 32, DNA (3: 1 g g⁻¹) | 20 | 13.8 |
| | 15 | 23.2 |
| | 10 | 33.1 |
| | 5 | 21.3 |

| | | |
|---|---|---|
| *% Expression relative to expression obtained for optimum DOTAP (DOTAP, β-gatactosidase reporter DNA ratio = 5: 1) formulation on dosing cells with 20µg DNA. % protein expression relative to DOTAP formulation obtained with 20µg naked DNA alone = 25.15% | | |

Transfection with DAB 8 is also slightly superior to that obtained with DOTAP (Table 2). This indicates a superior gene transfer activity for the DAB 8 and DAB 16 dendrimers. DAB 4 is currently being tested in our laboratories. For the DAB 16 formulation, transfection appears to be optimum when using DAB 16 complexes with DNA at a nitrogen to phosphate ratio of 30: 1, forming complexes of 150nm in size. Transfection with DAB 8 is also optimum at a nitrogen to phosphate ratio of 30: 1. Transfection with polyamidoamine dendrimers is optimum when low-density soluble material is formed at a nitrogen to phosphate ratio of 20:1¹⁹.

DAB 8 the most transfection efficient molecule studied to date in the polypropylenimine dendrimer class is also the least toxic, exhibiting an IC50 almost 6X higher than DOTAP (Table 3).

**Table 3: In vitro cytotoxicity against the A431 cell line**

| **Formulation** | **IC50 (µg ml⁻¹)** |
|---|---|
| DAB 8 | 352.4 |
| DAB 8, DNA (5: 1 g g⁻¹) | 669.4 |
| DAB 16 | 38.9 |
| DAB 16, DNA (5: 1gg⁻¹) | 36.0 |
| QDAB 16 | 44.6 |
| QDAB 16, DNA (3: 1gg⁻¹) | 129 |
| DAB 32 | 5.7 |
| DAB 32, DNA (3: 1 g g⁻¹) | 5.8 |
| QDAB 32 | 11.2 |
| QDAB 32 (3: 1g g⁻¹) | 33 |
| DOTAP | 62 |

The data in Table 3 indicate that the complex formed by DNA and the quatemised molecule (QDAB 32 and QDAB 16) is less toxic than that formed by DNA and the unquaternised molecule. The quartenised molecule QDAB 16 is as active as DOTAP at the 20µg DNA dose and QDAB 32 shows slight activity as a gene transfer agent at the 10µg dose level (Table 2) while DAB 32 is inactive. It is envisaged that QDAB 8 will produce a gene transfer formulation with good biocompatibility and also with no loss of activity when compared to the unquaternised parent polymer.

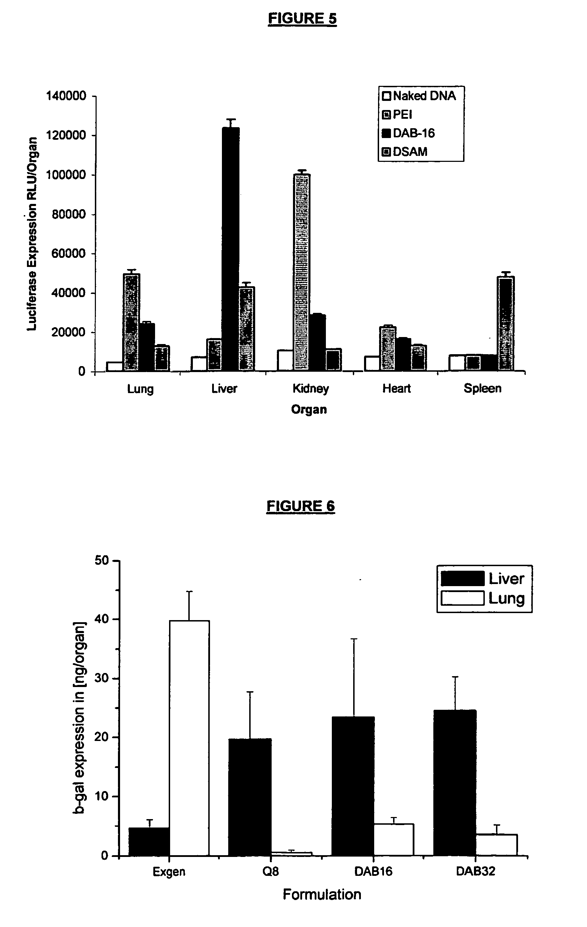

DAB 16 and DSAM are efficient deliverers of DNA to tissues in vivo comparing favourably to the commercial product Exgen 500 and with the added ability of being able to target the liver (DAB 16) and spleen (DSAM 16) more effectively than Exgen 500 (Table 4, Figure 5).

**Table 4: In vivo luciferase expression obtained in the mouse model**

| **Permutation** | **% Luciferase expression relative to Exgen 500*** | | | | |
|---|---|---|---|---|---|
| | **Lung** | **Liver** | **Kidney** | **Heart** | **Spleen** |
| DAB 16 | 48.9 | 762.6 | 28.6 | 73.2 | 100 |
| DSAM 16 | 25.8 | 264.3 | 11.3 | 58.2 | 599.2 |
| DNA Alone | 9.2 | 43.6 | 10.6 | 33.0 | 97.0 |

| | | | | | |
|---|---|---|---|---|---|
| * % luciferase expression relative to that obtained with linear PEI (Mw = 22kD, Exgen 500^{®}) on intravenous injection of 50µg DNA DAB 16 (DAB 16, DNA weight ratio = 5: 1) and DSAM 16 (DSAM 16, DNA weight ratio = 5: 1) formulations. Exgen 500 formulation consists of linear PEI, DNA weight ratio = 6:1. | | | | | |

### Conclusions

In summary the lower generation polypropylenimine dendrimers (DAB 8 and DAB 16) show improved biocompatibility when compared to DOTAP and transfection activity which is at some dose levels superior to that obtained with DOTAP. Additionally DAB 16 may be used to target the liver, and DSAM 16 used to target the spleen in vivo.

### EXAMPLE 2

### MATERIALS

All polypropylenimine dendrimers, glucose, were obtained from Sigma - Aldrich, UK. Phenyl methyl sulphonyl fluoride (PMSF), protease Inhibitor cocktail and phosphate buffered saline tablets, isopropanol and maltose were all supplied by Sigma Aldrich, UK. 9H-(1,3-dichloro-9,9- dimethylacridin-2-one-7-yl) -D-galactopyranoside (DDAO) was purchased from Molecular Probes . Exgen 500 (linear polyethylenimine, Mw = 22kD) was obtained from Euromedex, France. Passive lysis buffer was supplied by Promega, UK pCMV-beta gal DNA was obtained from Life Sciences/ Invitrogen and propagated in E.Coli as previously described⁹.

### METHODS

Groups of healthy female Balb-C mice (n = 3) were injected intravenously with either DAB8 - DNA, quaternary ammonium DAB8 (Q8) - DNA, DAB16 - DNA or DAB32 - DNA or Exgen 500 (linear polyethylenimine) - DNA. Formulations of the Dendrimer or Exgen 500, DNA Complexes (200µl) dispersed in glucose 5% w/v containing 100µg DNA were injected into each mouse and the dendrimer, DNA weight ratios were as follows: DAB8, Q8 and DAB 16 were all administered at a dendrimer, DNA weight ratio of 5: 1. DAB32 was administered at a dendrimer, DNA weight ratio of 3: 1. Exgen 500 was administered in accordance with the manufacturers instructions.
Mice were killed after 24h and their lungs and livers removed and frozen in liquid nitrogen until an assay for β-galactosidase could be performed. For the assay, 1g of organ was made up to 2mL with a protease lysis buffer. The protease lysis buffer consisted of a) Protease lysis buffer 5X (1mL), b) Phenyl methyl sulphonyl fluoride (PMSF) (50mM in methanol, 200µL), c) Protease Inhibitor cocktail (100µl) and water (3.7mL).

1 X 10⁶ A431 cells dispersed in 0.1 ml phosphate buffered saline (pH = 7.4, PBS) were implanted subcutaneously in each flank of CD-1 female nude mice. 4 days after the injection, the tumours were palpable (around 2 mm). 8 days after the injection, the size of the tumours increased (around 5 mm) and the blood vessels were more visible. The animals were dosed 11 days after tumour implantation. Mice (n=4) were injected intravenously with 50 □g DNA as naked DNA, DAB16 - DNA, Exgen500 - DNA all dispersed in 200 µL 5%w/v dextrose. Control animals were injected with 5%w/v dextrose. Animals were killed 24h later and tumours were excised and immediately frozen in liquid nitrogen. For the assay each tumour was added to 0.3mL of the Protease lysis buffer. Organs contained in the buffer were homogenised and 100 µl of the homogenised organ dispersion was added to 300 µl of the assay reagent. The assay reagent consisted of: a) DDAO 5 mg mL⁻¹ in DMSO (15µL), b) PMSF 50 mM in methanol (20µL), c) maltose 20 %w/v in PBS (100 µl), d) Protease inhibitor cocktail (15 µL), e) PBS (150 µl). The samples were incubated for the appropriate time (45-90 min.) at 37°C. 200 µl of this mixture was warmed at 95 °C for 2 min, in order to stop the β-galactosidase reaction and to denaturate the proteins which could interfere with the assay. 800 µl isopropanol was then added to the dispersion. The mixture obtained was vortexed to homogeneity and shaken for 20 min in the dark. The dispersion was then centrifuged for 4 min, at 13000 rpm. 500 µl of the supernatant was then added to 500 µl distilled water and the fluorescence read on a Beckman LS-50B fluorimeter (λ_{Exc}: 630 nm, λ_{Em} : 658 nm, slit: 2.5 nm). The amount of enzyme was then quantified using a β-galactosidase standard.

### RESULTS AND DISCUSSION

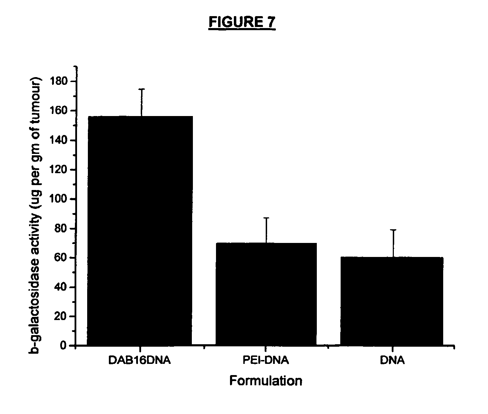

DAB16, Q8 and DAB 32 all resulted in liver targeting when compared to the commercial formulation Exgen500 (Figure 6). DAB 16 resulted in more gene expression in the tumours when compared to Exgen500 (Figure 7).

This data provides further support that polypropylenimine dendrimers target the liver and produce higher gene expression in tumour tissue when compared to commercial formulations where, for example, non-viral gene delivery systems target the lung^{8,53}. Targeting the liver is likely to prove useful in the treatment of liver enzyme deficiencies and liver tumours. Figure 7 illustrates that high expression in tumours may be obtained with the polypropylenimine dendrimers.

### REFERENCES

1. Anderson, W.F. (1998), Human gene therapy, Nature, 392, 25-30.
2. Verma, I.M. and Somia, N. (1997), Gene therapy - promises, problems and prospects, Nature, 389, 239-42.
3. Jolly, D., Viral vector systems for gene therapy, in The Internet Book of Gene Therapy, R.E. Sobol and K.J. Scanlon, Editors. 1996, Appleton & Lange. p. 3-25.
4. Felgner, P.L., Gadek, T.R., Holm, M., Roman, R., Chan, H.W., Wenz, M., Northrop, J.P., Ringold, G.M., and Danielsen, M. (1987), Lipofection - a Highly Efficient, Lipid-Mediated DNA-Transfection Procedure, Proc. Natl. Acad. Sci. USA, 84, 7413-7417.
5. Song, Y.K., Liu, F., Chu, S., and Liu, D. (1997), Characterization of cationic liposome-mediated gene transfer in vivo by intravenous administration, Human Gene Ther., 8, 1585-1594.
6. Song, Y.K., Liu, F., and Liu, D. (1998), Enhanced gene expression in mouse lung by prolonging the retention time of intravenously injected plasmid DNA, Gene Ther., 5, 1531-7.
7. Wagner, E., Zenke, M., Cotten, M., Beug, H., and Bimstiel, M.L. (1990), Transferrin-polycation conjugates as carriers for DNA uptake into cells, Proc. Natl. Acad. Sci. U. S. A., 87, 3410-4.
8. Ogris, M., Brunner, S., Schuller, S., Kircheis, R., and Wagner, E. (1999), Pegylated DNA/transferrin-PEI complexes: reduced interaction with blood components, extended circulation in blood and potential for systemic gene delivery, Gene Ther., 6, 595-605.
9. Brown, M.D., Schätzlein, A.G., Brownlie, A., Jack, V., Tetley, L., Gray, A.I., and Uchegbu, I. F. (2000), Preliminary characterisation of novel amino acid based polymeric vesicles as gene delivery agents, Bioconjug. Chem., 11, 880-891.
10. Brownlie, A., Gray, A.I., Schätzlein, A.G., Tetley, L., Tufail, U., and Uchegbu, I.F. (2000), Preliminary characterisation of modified polyethylenimine polymers for gene delivery, AAPS PharmSci, 2 (Suppl.), 2408.
11. Kukowska-Latallo, J.F., Bielinska, A.U., Johnson, J., Spindler, R., Tomalia, D.A., and Baker, J.R. (1996), Efficient Transfer of Genetic Material Into Mammalian-Cells Using Starburst Polyamidoamine Dendrimers, Proc. Natl. Acad. Sci. USA, 93, 4897-4902.
12. Tang, M.X., Redemann, C.T., and Szoka, F.C. (1996), In vitro gene delivery by degraded polyamidoamine dendrimers, Bioconjug. Chem., 7, 703-714.
13. Hudde, T., Rayner, S.A., Corner, R.M., Weber, M., Isaacs, J.D., Waldmann, H., Larkin, D.P.F., and George, A.J.T. (1999), Activated polyamidoamine dendrimers, a non-viral vector for gene transfer to the corneal endothelium, Gene Ther., 6, 939-943.
14. Loup, C., Zanta, M.A., Caminade, A.M., Majoral, J.P., and Meunier, B. (1999), Preparation of water-soluble cationic phosphorus-containing dendrimers as DNA transfecting agents, Chemistry-a Eur. J., 5, 3644-3650.
15. Debrabandervandenberg, E.M.M. and Meijer, E.W. (1993), Poly(Propylene Imine) Dendrimers - Large-Scale Synthesis by Hetereogeneously Catalyzed Hydrogenations, Angew. Chem. Intl. Ed. Engl., 32, 1308-1311.
16. Debrabandervandenberg, E.M.M., Nijenhuis, A., Mure, M., Keulen, J., Reintjens, R., Vandenbooren, F., Bosman, B., Deraat, R., Frijns, T., Vanderwal, S., Castelijns, M., Put, J., and Meijer, E.W. (1994), Large-Scale Production of Polypropylenimine Dendrimers, Macromolecul. Symp., 77, 51-62.
17. vanDuijvenbode, R.C., Borkovec, M., and Koper, G.J.M. (1998), Acid-base properties of poly(propylene imine) dendrimers, Polymer, 39, 2657-2664.
18. Bielinska, A.U., Yen, A., Wu, H.L., Zahos, K.M., Sun, R., Weiner, N.D., Baker, J.R., and Roessler, B.J. (2000), Application of membrane-based dendrimer/DNA complexes for solid phase transfection in vitro and in vivo, Biomaterials, 21, 877-887.
19. Bielinska, A.U., Chen, C.L., Johnson, J., and Baker, J.R. (1999), DNA complexing with polyamidoamine dendrimers: Implications for transfection, Bioconjug. Chem., 10, 843-850.
20. Toth, I., Sakthivel, T., Wilderspin, A.F., Bayele, H., Odonnell, M., Perry, D.J., Pasi, K.J., Lee, C.A., and Florence, A.T. (1999), Novel cationic lipidic peptide dendrimer vectors - In vitro gene delivery, STP Pharma Sci., 9, 93-99.
21. Du, B., Zhou, R.J., and Zhuo, R.X. (1998), Synthesis of cyclic core dendritic polymer and its usage as a vector for transferring foreign DNA into human cells, Chin. Chem. Lett., 9, 635-638.
22. Bielinska, A., Kukowskalatallo, J., Piehler, L.T., Yin, R., Spindler, R., Tomalia, D.A., and Baker, J.R. (1995), Starburst(R) Pamam Dendrimers - a Novel Synthetic Vector for the Transfection of DNA into Mammalian-Cells, Abstr. Pap. Am. Chem. Soc., 210, 145-PMSE.
23. Bielinska, A., Johnson, J., Kukowskalatallo, J., Tomalia, D., Spindler, R., and Baker, J. (1995), Unique Characteristics of Starburst(Tm) Dendrimers in in-Vitro DNA Transfer, Faseb J., 9, A312.
24. Bielinska, A.U., KukowskaLatallo, J.F., and Baker, J.R. (1997), The interaction of plasmid DNA with polyamidoamine dendrimers: mechanism of complex formation and analysis of alterations induced in nuclease sensitivity and transcriptional activity of the complexed DNA, Biochim. Biophys. Acta, 1353, 180-190.
25. BenMamoun, C., Truong, R., Gluzman, I., Akopyants, N.S., Oksman, A., and Goldberg, D.E. (1999), Transfer of genes into Plasmodium falciparum by polyamidoamine dendrimers, Mol. Biochem. Parasitol., 103, 117-121.
26. Pistolis, G., Malliaris, A., Tsiourvas, D., and Paleos, C.M. (1999), Poly(propyleneimine) dendrimers as pH-sensitive controlled-release systems, Chemistry-a Eur. J., 5, 1440-1444.
27. Sideratou, Z., Tsiourvas, D., and Paleos, C.M. (2000), Quatemized poly(propylene imine) dendrimers as novel pH-sensitive controlled-release systems, Langmuir, 16, 1766-1769.
28. Jansen, J., Debrabandervandenberg, E.M.M., and Meijer, E.W. (1994), Encapsulation of Guest Molecules into a Dendritic Box, Science, 266, 1226-1229.
29. Malik, N., Wiwattanapatapee, R., Klopsch, R., Lorenz, K., Frey, H., Weener, J.W., Meijer, E.W., Paulus, W., and Duncan, R. (2000), Dendrimers: Relationship between structure and biocompatibility in vitro, and preliminary studies on the biodistribution of 1-125- labelled polyamidoamine dendrimers in vivo Download Full Text of Article, J. Control. Rel. 65, 133-148.
30. Kabanov, V.A., Zezin, A.B., Rogacheva, V.B., Gulyaeva, Z.G., Zansochova, M.F., Joosten, J.G.H., and Brackman, J. (1999), Interaction of Astramol poly(propyleneimine) dendrimers with linear polyanions, Macromolecules, 32, 1904-1909.
31. Gebhart, C.L. and Kabanov, A.V. (2001), Evaluation of polyplexes as gene transfer agents, J. Control. Rel., 73, 401-406.
32. Zhang, X.Y., Wilhelm, M., Klein, J., Pfaadt, M., and Meijer, E.W. (2000), Modification of surface interactions and friction by adsorbed dendrimers: 1. Low surface-energy fifth-generation amino acid- modified poly(propyleneimine) dendrimers, Langmuir, 16, 3884-3892.
33. vanDuijvenbode, R.C., Rajanayagam, A., Koper, G.J.M., Baars, M., deWaal, B.F.M., Meijer, E.W., and Borkovec, M. (2000), Synthesis and protonation behavior of carboxylate-functionalized poly(propyleneimine) dendrimers, Macromolecules, 33, 46-52.
34. Roman, C., Fischer, H.R., and Meijer, E.W. (1999), Microphase separation of diblock copolymers consisting of polystyrene and acid-functionalized poly(propylene imine) dendrimers, Macromolecules, 32, 5525-5531.
35. Bodnar, I., Silva, A.S., Deitcher, R.W., Weisman, N.E., Kim, Y.H., and Wagner, N.J. (2000), Structure and rheology of hyperbranched and dendritic polymers. I. Modification and characterization of poly(propyleneimine) dendrimers with acetyl groups, J. Polymer Sci. Part B-Polymer Phys., 38, 857-873.
36. Chen, C.Z., Tan, N.C.B., and Cooper, S.L. (1999), Incorporation of dimethyldodecylammonium chloride functionalities onto poly(propylene imine) dendrimers significantly enhances their antibacterial properties, Chem. Commun., 1585-1586.
37. Baars, M., Kleppinger, R., Koch, M.H.J., Yeu, S.L., and Meijer, E.W. (2000), The localization of guests in water-soluble oligoethyleneoxy-modified poly(propylene imine) dendrimers, Angew. Chem.-Intl. Ed., 39, 1285 -1290.
38. Peerlings, H.W.l., Nepogodiev, S.A., Stoddart, J.F., and Meijer, E.W. (1998), Synthesis of spacer-armed glucodendrimers based on the modification of poly(propylene imine) dendrimers, Eur. J. Org. Chem., 1879-1886.
39. Pavlov, G.M., Komeeva, E.V., Jumel, K., Harding, S.E., Meijer, E.W., Peerlings, H.W.I., Stoddart, J.F., and Nepogodiev, S.A. (1999), Hydrodynamic properties of carbohydrate-coated dendrimers, Carbohydrate Polymers, 38, 195-202.
40. Cameron, J.H., Facher, A., Lattermann, G., and Diele, S. (1997), Poly(propyleneimine) dendromesogens with hexagonal columnar mesophase, Advanced Materials, 9, 398 -405.
41. Schenning, A., ElissenRoman, C., Weener, J.W., Baars, M., vanderGaast, S.J., and Meijer, E.W. (1998), Amphiphilic dendrimers as building blocks in supramolecular assemblies, J. Am. Chem. Soc., 120, 8199-8208.
42. Liu, M.J. and Frechet, J.M..J. (1999), Preparation, MALDI-TOF analysis, and micelle-like behavior of alkyl- modified poly(propylene imine) dendrimers, Polymer Bull., 43, 379-386.
43. Liu, M.J., Petro, M., Frechet, J.M.J., Haque, S.A., and Wang, H.C. (1999), Preparation of hydrophobic poly(isobutylene) star polymers with hydrophilic poly(propylene imine) dendritic cores, Polymer Bull., 43, 51-58.
44. Froehling, P.E. and Linssen, H.A.J. (1998), Positional and compositional heterogeneity of partially modified poly(propyleneimine) dendrimers, Macromolecul. Chem. Phys., 199, 1691-1695.
45. Yonetake, K., Masuko, T., Morishita, T., Suzuki, K., Ueda, M., and Nagahata, R. (1999), Poly(propyleneimine) dendrimers peripherally modified with mesogens, Macromolecules, 32, 6578-6586.
46. Schenning, A., Peeters, E., and Meijer, E.W. (2000), Energy transfer in supramolecular assemblies of oligo(p-phenylene vinylene)s terminated poly(propylene imine) dendrimers, J. Am. Chem. Soc., 122, 4489-4495.
47. Ausubel, F., Brent, R., Kingston, E., Moore, D., Seidman, J., Smith, J., and Struhl, K., eds. Current Protocols In Molecular Biology., ed. V. Chanda . Vol. I-III. 1998, John Wiley & Sons.
48. Pecq, J.-B.L. (1971), Use of ethidium bromide for separation and determination of nucleic acids of various conformational forms and measurement of their associated enzymes, Methods Biochem Anal, 20, 41-86.
49. Freshney, R.I., Culture of animal cells : a manual of basic technique. 3rd ed. 1994, New York: Wiley-Liss. xxiv, 486.
50. Lee, R.J., Wang, S., and Low, P.S. (1996), Measurement of endosome pH following folate receptor-mediated endocytosis, Biochim. Biophys. Acta , 1312, 237-242.
51. Bosman, A.W., Janssen, H.M., Meijer, E.W. (1999). About dendrimers: structure, physical properties, and applications. Chem. Rev. 99, 1665-1688.
52. De Brabander-van den Berg, E.M.M., Meijer, E.W. (1993). Poly(propylene imine) dendrimers: large scale synthesis by heterogenously catalyzed hydrogenations. Angew. Chem. Int. Ed. Engl. 32, 1308-1311.
53. M.D. Brown, A. Schätztein, A. Brownlie, V. Jack, W. Wang, L. Tetley, A.I. Gray, I.F. Uchegbu, Preliminary characterization of novel amino acid based polymeric vesicles as gene and drug delivery agents, Bioconjugate Chemistry, 11 (2000), 880-91.

## Claims

1. A composition for the delivery of a bioactive molecule to a target location in a body of a recipient, said composition comprising a cationic polypropylenimine dendrimer comprising a diaminobutane core with 1, 2 or 3 generations of propylenimine molecules attached which is admixed with the bioactive molecule, wherein the cationic dendrimer is optionally modified by covalently binding derivatising groups.

2. The composition according to claim 1, wherein the cationic dendrimer is derivatised using groups selected from the group consisting of hydrophobic, hydrophilic and amphiphilic groups.

3. The composition according to claim 1, wherein the cationic dendrimer is derivatised by binding two dendrimer molecules to either end of a hydrocarbon chain.

4. The composition according to claim 3, wherein the length of the hydrocarbon chain is selected from the group consisting of 8, 12, 14, 16 and 18 carbon atoms.

5. The composition according to claim 3, or claim 4, wherein the derivatised cationic dendrimer is a bolamphiphilic dendrimer.

6. The composition according to claim 5, wherein the number of derivatising groups is from one derivatising group per dendrimer molecule up to and including derivatising all available surface or terminal groups on the dendrimer molecule.

7. The composition according to claim 2, wherein the amphiphilic derivative comprises a hydrophilic and hydrophobic segment.

8. The composition according to claim 2, wherein the hydrophilic segment is derived from a phosphoglycerate molecule.

9. The composition according to claim 7, wherein the hydrophobic segment is covalently bound to the hydrophilic segment via an ester linkage.

10. The composition according to claim 7 or claim 9, wherein the hydrophobic segment is selected from the group consisting of alkyl, alkenyl or alkynyl groups of from 8 to 24 carbons in length.

11. The composition according to claim 7, wherein the amphiphilic derivative is attached to the dendrimer by a linker molecule selected from the group consisting of polyethylene glycol (PEG) and a sugar molecule.

12. The composition according to claim 11, wherein the length of the PEG linker molecule is from 1 to 120 ethylene glycol units.

13. The composition according to claim 11, wherein the linker molecule is a phosphoglyceride.

14. The composition according to claim 7, wherein the number of amphiphilic derivatives per dendrimer molecule is from one derivatising group per dendrimer molecule to derivatising all of the groups of the dendrimer.

15. The composition according to claim 1, wherein the target location in the body of the recipient is selected from the group consisting of the liver, spleen, lung, kidney and heart.

16. The composition according to claim 15, wherein the composition is for the delivery of a bioactive molecule to the liver of a recipient, and wherein said composition comprises the polypropylenimine dendrimer DAB16 admixed with a said bioactive molecule.

17. The composition according to claim 15, wherein the composition is for the delivery of a bioactive molecule to the spleen of a recipient, and wherein said composition comprises the polypropylenimine dendrimer DSAM16 admixed with a said bioactive molecule.

18. The composition according to any one of the preceding claims, wherein the composition is suitable for delivery to a human recipient.

19. The composition according to any one of the preceding claims, wherein the bioactive molecules are selected from the group consisting of polynucleotides, peptides, polypeptides and pharmaceutically active agents.

20. A composition for *in vitro* transfection of mammalian cells with a bioactive molecule said composition comprising a cationic polypropylenimine dendrimer comprising a diaminobutane core with 1, 2 or 3 generations of propylenimine molecules attached which is admixed with the bioactive molecule, wherein the cationic dendrimer is optionally modified by covalently binding derivatising groups.

21. A pharmaceutical formulation comprising a composition as described in any one of claims 1 to 19, and a pharmaceutically acceptable carrier.

22. Use of the composition according to any one of claims 1 to 19 for the preparation of a medicament for the delivery of said bioactive molecule to a target location in the body of a recipient.

23. Use of a composition according to claim 22, wherein the target location is selected from the group consisting of the liver, spleen, lung, kidney and heart.

## Patentansprüche

1. Zusammensetzung zur Zufuhr eines bioaktiven Moleküls zu einem Zielort im Körper eines Rezipienten, wobei die Zusammensetzung ein kationisches Polypropylenimindendrimer umfasst, das einen Diaminobutan-Kern mit 1, 2 oder 3 daran gebundenen Generationen von Propyleniminmolekülen aufweist und mit dem bioaktiven Molekül gemischt ist, wobei das kationische Dendrimer gegebenenfalls durch die kovalente Bindung von derivatisierenden Gruppen modifiziert ist.

2. Zusammensetzung nach Anspruch 1, worin das kationische Dendrimer unter Einsatz von den aus der aus hydrophoben, hydrophilen und amphiphilen Gruppen bestehenden Gruppe ausgewählten Gruppen derivatisiert ist.

3. Zusammensetzung nach Anspruch 1, worin das kationische Dendrimer durch die Bindung von zwei Dendrimermolekülen an beide Enden einer Kohlenwasserstoffkette derivatisiert ist.

4. Zusammensetzung nach Anspruch 3, worin die Länge der Kohlenwasserstoffkette aus der aus 8, 12, 14, 16 und 18 Kohlenstoffatomen bestehenden Gruppe ausgewählt ist.

5. Zusammensetzung nach Anspruch 3 oder 4, worin das derivatisierte kationische Dendrimer ein bolaamphiphiles Dendrimer ist.

6. Zusammensetzung nach Anspruch 5, worin die Anzahl der derivatisierenden Gruppen eine derivatisierende Gruppe pro Molekül bis zu und einschließlich Derivatisieren aller verfügbaren Oberflächen- oder terminalen Gruppen an dem Dendrimermolekül beträgt.

7. Zusammensetzung nach Anspruch 2, worin das amphiphile Derivat ein hydrophiles und ein hydrophobes Segment umfasst.

8. Zusammensetzung nach Anspruch 2, worin das hydrophile Segment von einem Phosphoglycerat-Molekül stammt.

9. Zusammensetzung nach Anspruch 7, worin das hydrophobe Segment über eine Esterbindung kovalent an das hydrophile Segment gebunden ist.

10. Zusammensetzung nach Anspruch 7 oder 9, worin das hydrophobe Segment aus der aus Alkyl-, Alkenyl- oder Alkinylgruppen mit 8 bis 24 Kohlenstoffatomen bestehenden Gruppe ausgewählt ist.

11. Zusammensetzungen nach Anspruch 7, worin das amphiphile Derivat durch ein Linkermolekül, das aus der aus Polyethylenglykol (PEG) und einem Zuckermolekül bestehenden Gruppe ausgewählt ist, an das Dendrimer gebunden ist.

12. Zusammensetzung nach Anspruch 11, worin die Länge des PEG-Linkermoleküls 1 bis 120 Ethylenglykol-Einheiten beträgt.

13. Zusammensetzung nach Anspruch 11, worin das Linkermolekül ein Phosphoglycerid ist.

14. Zusammensetzung nach Anspruch 7, worin die Anzahl der amphiphilen Derivate pro Dendrimermolekül eine derivatisierende Gruppe pro Dendrimermolekül bis zu Derivatisieren aller Gruppen des Dendrimers beträgt.

15. Zusammensetzung nach Anspruch 1, worin der Zielort im Körper des Rezipienten aus der aus Leber, Milz, Lunge, Nieren und Herz bestehenden Gruppe ausgewählt ist.

16. Zusammensetzung nach Anspruch 15, worin die Zusammensetzung zur Zufuhr eines bioaktiven Moleküls zur Leber des Rezipienten dient und worin die Zusammensetzung das Polypropylenimindendrimer DAB16 gemischt mit dem bioaktiven Molekül umfasst.

17. Zusammensetzung nach Anspruch 15, worin die Zusammensetzung zur Zufuhr eines bioaktiven Moleküls zur Milz des Rezipienten dient und worin die Zusammensetzung das Polypropylenimindendrimer DSAM16 gemischt mit dem bioaktiven Molekül umfasst.

18. Zusammensetzung nach einem der vorangegangenen Ansprüche, worin die Zusammensetzung für die Verabreichung an einen menschlichen Rezipienten geeignet ist.

19. Zusammensetzung nach einem der vorangegangenen Ansprüche, worin die bioaktiven Moleküle aus der aus Polynucleotiden, Peptiden, Polypeptiden und pharmazeutisch annehmbaren Mitteln bestehenden Gruppe ausgewählt sind.

20. Zusammensetzung zur In-vitro-Transfektion von Säugetierzellen mit einem bioaktiven Molekül, wobei die Zusammensetzung ein kationisches Polypropylenimindendrimer umfasst, das einen Diaminobutan-Kern mit 1, 2 oder 3 Generationen daran gebundener Propyleniminmoleküle aufweist und das mit dem bioaktiven Molekül gemischt ist, worin das kationische Dendrimer gegebenenfalls durch die kovalente Bindung von derivatisierenden Gruppen modifiziert ist.

21. Pharmazeutische Formulierung, umfassend eine Zusammensetzung wie in den Ansprüchen 1 bis 19 beschrieben und einen pharmazeutisch annehmbaren Träger.

22. Verwendung der Zusammensetzung nach einem der Ansprüche 1 bis 19 zur Herstellung eines Medikaments zur Zufuhr des bioaktiven Moleküls zu einem Zielort im Körper eines Rezipienten.

23. Verwendung einer Zusammensetzung nach Anspruch 22, worin der Zielort aus der aus Leber, Milz, Lunge, Nieren und Herz bestehenden Gruppe ausgewählt ist.

## Revendications

1. Composition pour la délivrance d'une molécule bio-active en un emplacement cible dans le corps d'un récepteur, ladite composition comprenant un dendrimère polypropylèneimine cationique, comprenant un corps diaminobutane avec 1, 2 ou 3 générations de, molécules propylèneimine attachées, qui est mélangé à la molécule bio-active, où le dendrimère cationique est le cas échéant, modifié par liaison covalente de groupements de dérivatisation.

2. Composition selon la revendication 1, dans laquelle le dendrimère cationique est dérivatisé avec des groupements choisis parmi le groupe consistant en des groupements hydrophobes, hydrophiles et amphiphiles.

3. Composition selon la revendication 1, dans laquelle le dendrimère cationique est dérivatisé par liaison de deux molécules de dendrimère sur chaque extrémité d'une chaîne hydrocarbonée.

4. Composition selon la revendication 3, dans laquelle la longueur de la chaîne hydrocarbonée est choisie parmi le groupe consistant en 8, 12, 14, 16 ou 18 atomes de carbone.

5. Composition selon la revendication 3 ou 4, dans laquelle le dendrimère cationique dérivatisé est un dendrimère bolamphiphile.

6. Composition selon la revendication 5, dans laquelle le nombre de groupements de dérivatisation est de un groupement de dérivatisation par molécule dendrimère à et y compris la dérivatisation de toute la surface disponible ou les groupements terminaux sur la molécule dendrimère.

7. Composition selon la revendication 2, dans laquelle le dérivé amphiphile comprend un segment hydrophile et un segment hydrophobe.

8. Composition selon la revendication 2, dans laquelle le segment hydrophile dérive d'une molécule phosphoglycérate.

9. Composition selon la revendication 7, dans laquelle le segment hydrophobe est lié de manière covalente au segment hydrophile via une liaison ester.

10. Composition selon la revendication 7 ou 9, dans laquelle le segment hydrophobe est choisi parmi le groupe consistant en des groupements alkyle, alcényle ou alcynyle de 8 à 24 carbones de long.

11. Composition selon la revendication 7, dans laquelle le dérivé amphiphile est lié au dendrimère par une molécule lieur choisie parmi le groupe consistant en le polyéthylèneglycol (PEG) et une molécule de sucre.

12. Composition selon la revendication 11, dans laquelle la longueur de la molécule lieur PEG est de 1 à 120 unités éthylèneglycol.

13. Composition selon la revendication 11, dans laquelle la molécule lieur est un phosphoglycéride.

14. Composition selon la revendication 7, dans laquelle le nombre de dérivés amphiphiles par molécule dendrimère se situe depuis un groupement de dérivatisation par molécule dendrimère à la dérivatisation de tous les groupements du dendrimère.

15. Composition selon la revendication 1, dans laquelle l'emplacement cible dans le corps du récepteur est choisi parmi le groupe consistant en le foie, la rate, le poumon, le rein et le coeur.

16. Composition selon la revendication 15, dans laquelle la composition est destinée à la délivrance d'une molécule bio-active au foie du récepteur, et dans laquelle ladite composition comprend le dendrimère polypropylèneimine DAB16 mélangé à ladite molécule bio-active

17. Composition selon la revendication 15, dans laquelle la composition est destinée à la délivrance d'une molécule bio-active à la rate du récepteur, et dans laquelle ladite composition comprend le dendrimère polypropylèneimine DSAM16 mélangé à ladite molécule bio-active

18. Composition selon l'une quelconque des revendications précédentes, dans laquelle la composition est appropriée pour une délivrance à un récepteur humain.

19. Composition selon l'une quelconque des revendications précédentes, dans laquelle les molécules bio-actives sont choisies parmi le groupe consistant en des polynucléotides, des peptides, des polypeptides et des agents pharmaceutiquement actifs.

20. Composition pour la transfection *in vivo* de cellules mammaliennes avec une molécule bio-active, ladite composition comprenant un dendrimère polypropylèneimine cationique, comprenant un corps diaminobutane avec 1, 2 ou 3 générations de molécules propylèneimine attachées, qui est mélangé à la molécule bio-active, où le dendrimère cationique est le cas échéant, modifié par liaison covalente de groupements de dérivatisation.

21. Formulation pharmaceutique comprenant une composition, telle que décrite dans l'une quelconque des revendications 1 à 19, et un support pharmaceutiquement acceptable.

22. Utilisation de la composition selon l'une quelconque des revendications 1 à 19, pour la préparation d'un médicament pour la délivrance de ladite molécule bio-active en un emplacement cible dans le corps d'un récepteur.

23. Utilisation d'une composition selon la revendication 22, dans laquelle l'emplacement cible est choisi parmi le groupe consistant en le foie, la rate, le poumon, le rein et le coeur.
